# EUROPEAN PATENT APPLICATION

(11) **EP 4 455 672 A1**
(43) Date of publication of application: **30.10.2024**
(21) Application number: 23315115.8
(22) Date of filing: 26.04.2023
(51) Int. Cl.: G01N 33/68

(54) **METHOD AND KIT FOR DIAGNOSING AND MONITORING CHRONIC GRAFT-VERSUS-HOST DISEASE (CGVHD) USING PARTICULAR CYTOKINE BIOMARKERS**

(71) Applicant: Medsenic, 67100 Strasbourg (FR)
(72) Inventor: Rieger, François, 1203 GENEVE (CH); Nicco, Carole, 91440 BURES-SUR-YVETTE (FR); Rongvaux-Gaïda, Dominique, 67190 STILL (FR)
(74) Representative: Santarelli

(57) **Abstract**

The present disclosure relates to a method and kit for diagnosing and monitoring chronic graft-versus-host disease (cGVHD) in an individual who has received an allogeneic hematopoietic stem cell transplantation (allo-HSCT) and which can be used for determining if a patient responds to a treatment for cGVHD. This method consists in measuring the cytokines GM-CSF, CCL4, IL-1α, IL-17A, IL-21 and CXCL1 in a biological sample from the individual.

## Description

### FIELD OF THE INVENTION

The present I Invention belongs to the medical domain and more particularly to the diagnosis, prognosis, stratification and monitoring of chronic graft-vs-host disease (cGVHD).

Indeed, the present Invention concerns a method for the diagnosis, prognosis, stratification and monitoring of cGVHD, based on the detection of particular cytokines in biological samples from individuals who have received allogeneic hematopoietic stem cell transplantation (allo-HSCT).

The present invention also concerns a kit implemented in such a method.

### BACKGROUND OF THE INVENTION

### Allogenic hematopoietic stem cell transplantation (allo-HSCT) and Graft-Vs-Host Disease (GVHD)

Allogeneic hematopoietic stem cell transplantation (allo-HSCT) consists in the transplantation of multipotent hematopoietic stem cells in a subject, where such cells replicate and produce healthy normal blood cells.

For this reason, allo-HSCT is primarily used as a curative treatment of both malignant and non-malignant hematological disorders such as various cancers of the blood and of the bone marrow, such as multiple myeloma or leukemia.

Although its current use in the treatment of these diseases and a number of clinical trials currently ongoing to set up treatments for many other malignant and non-malignant hematological disorders (Yaniv I *et al.* 2018; Mahmoud HK *et al.* 2015), a major long-term complication of allo-HSCT is graft-vs-host disease (GVHD).

GVHD is a systemic disorder that occurs when the immune cells of the graft of the donor recognize the host as foreign and attack the recipient's body cells. GVHD is unique to allogeneic transplantation, *i.e.* to transplantation performed using the stem cells of a donor.

Unless the patient's donor is an identical twin, a patient receiving an allogeneic stem cell transplant will receive some type of treatment to prevent GVHD. This may include removing T-cells from the donor graft and/or giving medications to suppress the T-cells in the graft so that they do not attack the patient's cells. There is no standard regimen for the prevention of GVHD, and different combinations of medications are given at different institutions, with a classical standard of care, mainly involving the administration of corticosteroids (CS).

### Classification and current treatments of GVHD

GVHD is classified as acute graft-versus-host disease (aGVHD) and chronic graft-versus-host disease (cGVHD).

Whether acute or chronic, GVHD occurs in 20-50% of cases (Lee SJ et *al.* 2018) and remains the most common complication after transplantation (Staffas A *et al.* 2017).

The acute form usually develops within the first 100 days after transplantation. Acute GVHD can affect the skin, the gastrointestinal tract or the liver. The treatment of acute GVHD depends on the severity of the form but it usually consists, whether it is a mild form restricted, for example, to the skin, a severe and/or a systemic (or "whole-system") manifestation, in the administration of corticosteroids (CS) and/or cyclosporine A (CsA) (Rongvaux-Gaida *et al.* 2022). Nowadays, many patients who develop acute GVHD in one of its possible manifestations are treated with increased immunosuppression in the form of CS.

Chronic GVHD is a syndrome of variable clinical features resembling autoimmune and other immunologic disorders, such as scleroderma, Sjögren's syndrome, primary biliary cirrhosis, wasting syndrome, bronchiolitis obliterans, immune cytopenias and chronic immunodeficiency (Jagasia *et al.* 2015). Clinical manifestations nearly always present during the first year after transplantation, but some cases develop many years later (Jagasia *et al.* 2015). Manifestations of chronic GVHD may be restricted to a single organ or site but are usually widespread, with profound impact on the quality of life (Jagasia *et al.* 2015).

It should be noted that, during the development of cGVHD, there can be a period of time during which the acute and chronic forms of GVHD coexist. In this case, the acute and chronic forms are present at the same time in the same individual and independently of each other.

Generally speaking, the treatment for acute GVHD usually starts with CS. CS can be administered alone or more often in combination with anti-graft rejection agents, such as calcineurin inhibitors.

Concerning the chronic form, cGvHD, patients with mild symptoms, especially if the symptoms are limited to a single organ or site, can often be treated with local/topical symptomatic therapies. Patients with more severe symptoms or multi organ involvement typically require "systemic" immunosuppressive treatments. Prednisone is the standard first-line therapy for cGVHD. For patients who do not respond to steroid treatment, there are second-line treatments. Ibrutinib (Imbruvica) is an FDA-approved drug for the treatment of adult patients with cGVHD after failure of one or more lines of systemic therapy. Other treatments include the administration of mycophenolate mofetil, sirolimus, tacrolimus or cyclosporine, monoclonal antibodies such as infliximab (Remicade), tocilizumab (Actemra), alemtuzumab (Campath), basiliximab (Simulect), daclizumab (Zinbryta) and denileukin diftitox (Ontak), antithymocyte globulin (ATG), pentostatin (Nipent) and ruxolitinib (Jakafi). Arsenic Trioxide (ATO) is also used for the treatment of cGVHD. In addition, extracorporeal photopheresis (ECP) is being developed for the treatment of cGVHD, as this therapy has the advantage of not increasing infectious risks (Hart JW *et al.* 2013).

Concerning the treatments, it has to be noted that, despite their widespread use, CS have far from optimal response rates, with 40% to 60% of transient responses for cGVHD patients (Fowler DH *et al.* 2015; Dignan FL *et al*. 2012). Furthermore, prolonged use of CS, despite being the rule, is associated with increasingly serious side effects, such as malignancy relapse, infections, myopathy, cataracts, hyperglycemia, decline in bone mass and avascular necrosis (Saidu NE *et al.* 2020). For these reasons, new agents with a better safety profile and higher efficacy are urgently needed.

It is also important to note that, while the mechanisms that cause the inflammation and tissue damage of aGVHD are now quite well understood, the pathobiology of cGVHD is more complex and less well understood. Many investigators believe that the destructive immunological and autoimmune mechanisms that cause cGVHD are quite distinct from aGVHD, irrespective of whether or not the cGVHD evolves from acute.

The lack of effective treatments for cGVHD is even more serious in light of the fact that cGVHD is a debilitating and life-threatening disease in its moderate to severe forms. cGVHD continues to account for significant morbidity and mortality in the outcome of patients undergoing allo-HSCT. Although improvements have been made in the prevention of aGVHD through better genetic choices in donors, these advances have not resulted in a concomitant decrease in the incidence of cGVHD (Lee SJ *et al.* 2015). On the contrary, the prevalence of cGVHD has increased over the past 20 years (Socié G. *et al.* 2014).

Because of the need to be able to track the effects of new treatments for cGVHD, and for other reasons explained below, the present Invention relates specifically to the chronic form of GVHD.

### Current diagnostic standards for cGVHD and their limitations

Not only, as explained above, cGVHD can be difficult to treat, but it also poses a challenge in terms of diagnosis.

In fact, historically, diagnosis and scoring of cGVHD have been difficult because of pleiotropic organ manifestations and heterogeneous diagnostic criteria. A major advancement in the field was the development of the National Institutes of Health (NIH) consensus criteria to define a clinical disease model and framework that could be rigorously applied to clinical studies. The revised 2015 NIH criteria have brought much-needed consistency to terminology and methods for disease diagnosis and staging (Jagasia *et al.* 2015).

The NIH established a scoring system based on a 0-3 scale that describes the extent and severity of cGVHD for each organ or site based on consensus criteria. cGVHD is classified as mild, moderate or severe, based on the number and severity of the diseases in the affected organs.

Mild cGVHD involves 2 or fewer organs with no more than score 1 and no lung involvement. Mild cGVHD is associated with a good prognosis and is generally treated with topical or local therapies, although systemic therapy may sometimes be required for patients presenting high risk features such as thrombocytopenia and hyperbilirubinemia. Patients with mild or asymptomatic manifestations limited to a single organ or site can often be managed with topical treatment or by slowing the taper of prophylactic immunosuppressive treatment (Jagasia *et al.* 2015).

Moderate disease is assigned with score 1 either if only the lung is concerned or if 3 or more organs are involved and with score 2 if any organ is involved.

Severe disease is assigned with score 2 or 3 if only the lung is concerned and with score 3 if at least one organ is affected, and means that substantial organ damage already exists. Moderate to severe cGVHD usually requires systemic immunosuppressive treatment, with the most severe cases being associated with higher treatment-related mortality and lower survival rate.

Despite the recent advances in the diagnosis of cGVHD, diagnosing and scoring the severity of chronic GVHD is challenging for several reasons:
- the limited understanding of the pathophysiology (Jagasia *et al.* 2015);
- the current scoring metrics for cGVHD are frequently dependent on external symptoms such as fever and skin rashes and do not have the required objectivity;
- the current diagnosis methods do not have specificity for cGVHD, and distinguishing the disease from other disorders such as Sjogren's syndrome and scleroderma, remains difficult. In addition, the coexistence of acute GVHD manifestations also pose problems to the diagnosis (Jagasia *et al.* 2015);
- the lack of suitable biomarkers for the diagnosis and assessment of the disease (Jagasia *et al.* 2015).

### The existing gap of knowledge about cGVHD

From the above, it emerges that cGVHD represents a serious disease whose biology is still not well-understood and whose diagnosis and treatment are still far from being optimal. In summary:
- The pathobiology of cGVHD is complex, not well understood and many investigators believe that the mechanisms that cause cGVHD are radically distinct from aGVHD;
- Despite the recent advances in the diagnosis of cGVHD, the current diagnostic standards for cGVHD are frequently dependent on external symptoms and do not have the required objectivity. In addition, current diagnosis methods do not have specificity for cGVHD, and distinguishing the disease from other disorders remains difficult;

- cGVHD is a debilitating and life-threatening disease in its moderate to severe forms. cGVHD continues to account for significant morbidity and mortality in the outcome of patients undergoing allo-HSCT. The prevalence of cGVHD has been increasing over the past 20 years;
- CS have far from optimal response rates and their prolonged use is associated with important side effects, which prompt the search for new agents with a better safety profile and higher efficacy.

A consequence of the current non-optimal methods and metrics for the diagnosis of cGVHD is the possible delay in starting or adapting the treatment in a patient with cGVHD, which can result in a life-threatening course of the disease, with systemic clinical worsening. Thus, how to diagnose cGVHD in clinical cases and characterize the intensity of the disease is a critical issue that influences the long-term success of allogeneic cell transplantation.

Therefore, the present inventors aim to solve the technical problem existing in the clinical practice of being unable to early detect cGVHD and to stratify the degree of severity of the disease, in order to provide a way to intervene in a timely manner on the patients with an effective therapy.

In other words, the present inventors aim to propose a method allowing to predict the probability of cGVHD activation, to monitor its progression and/or to estimate the risk of clinical worsening.

### SUMMARY OF THE INVENTION

The present invention enables the purpose set by the inventors to be reached.

Indeed, the inventors unexpectedly discovered that the temporal concentration profiles of certain cytokines and chemokines allow to predict the likelihood of insurgence of cGVHD and monitor its progression.

As described in the experimental part below, the inventors found that, in a totally unexpected manner, the concentration profiles of some of the measured cytokines correlated with the presence (or absence) of cGVHD in the individuals enrolled in the clinical trial.

More particularly, the inventors investigated the temporal concentration profile of a number of cytokines in biological samples taken from individuals enrolled in a phase 2 recent clinical trial to evaluate the efficacy and safety of a first-line combination of ATO and corticosteroid (CS) treatment in adult patients with cGVHD requiring systemic therapy after first allo-HSCT for a hematologic disease (NCT02966301).

It should be noted that, at the time of filing of the present Application, no use of a specific and sensitive biomarker and no method for the diagnosis of cGVHD implementing such a biomarker was available.

According to a first aspect, the present Invention thus pertains to an *in vitro* method for the diagnosis, prognosis, stratification and/or monitoring of chronic graft-versus-host disease in an individual who has received an allogeneic hematopoietic stem cell transplantation (allo-HSCT), comprising the steps of:
a) measuring the level of at least one cytokine selected from the group consisting of granulocyte-macrophage colony-stimulating factor (GM-CSF) and macrophage inflammatory protein (CCL4 or MIP-1β) in a biological sample from said individual,
b) optionally, measuring the level of at least one cytokine selected from the group consisting of Interleukin-1 alpha (IL-1α), Interleukin-17A (IL-17A), Interleukin-21 (IL-21) and chemokine (C-X-C motif) ligand 1 (CXCL1), in a biological sample from said individual,
c) comparing each of the levels measured in a) and, optionally, in b), to a reference value,
wherein levels in the biological sample higher than the reference values are indicative for the presence and/or aggravation of cGVHD.

According to a specific embodiment, in step a) the levels of GM-CSF and CCL4 are measured.

According to some embodiments, the reference value can be (i) an 'internal' value taken from a previous sample from the same individual, (ii) an 'internal' value measured from a previous sample from the same individual prior to allo-HSCT, (iii) an 'internal' value measured from a previous sample from the same individual before or after the onset of cGVHD, or it can be (iv) an 'external' value measured from a cohort of healthy individuals. Therefore, according to the present Invention, the reference value can be an 'internal' or 'external' value, depending on whether it has been obtained from the same individual or from a different individuals or group of individuals (e.g., a study cohort), respectively, and it can be measured from a biological sample either before the allo-HSCT or before or after the onset of cGVHD.

The present Invention also pertains to the use of GM-CSF and/or CCL4, alone or combined to one or several cytokines selected from the group consisting of IL-1α, IL-17A, IL-21 and CXCL1, as a biomarker for the diagnosis, prognosis, stratification and/or monitoring of cGVHD in a subject who has received an allo-HSCT.

The Invention-also relates to a theranostic method for determining if an individual responds to a treatment of cGVHD, comprising measuring the level of one or more cytokines at different time points as described above, wherein a decrease in the level of one or more of said cytokines indicates that the individual responds to the treatment.

The present Invention also pertains to a method of treating cGVHD in an individual in need thereof, comprising the steps of:
a) measuring the levels of the cytokines according to the preceding aspects in biological samples taken from the individual at different time points;
b) administering a medication for treating cGVHD such as corticosteroid (CS) and/or ATO to the individual; and
c) adapting the dosage regimen of said medication(s) for treating cGVHD such as CS and/or ATO depending on the evolution of the levels of said cytokines, wherein the dosage is tapered down if the levels decrease and can be tapered off if the levels become inferior to normal reference values.

A kit of parts for the diagnosis, prognosis, stratification and/or monitoring of cGVHD using a biological sample taken from an individual, and comprising reagents for measuring the level of one or more cytokines at different time points as described above, is also part of the present Invention.

### FIGURE LEGENDS

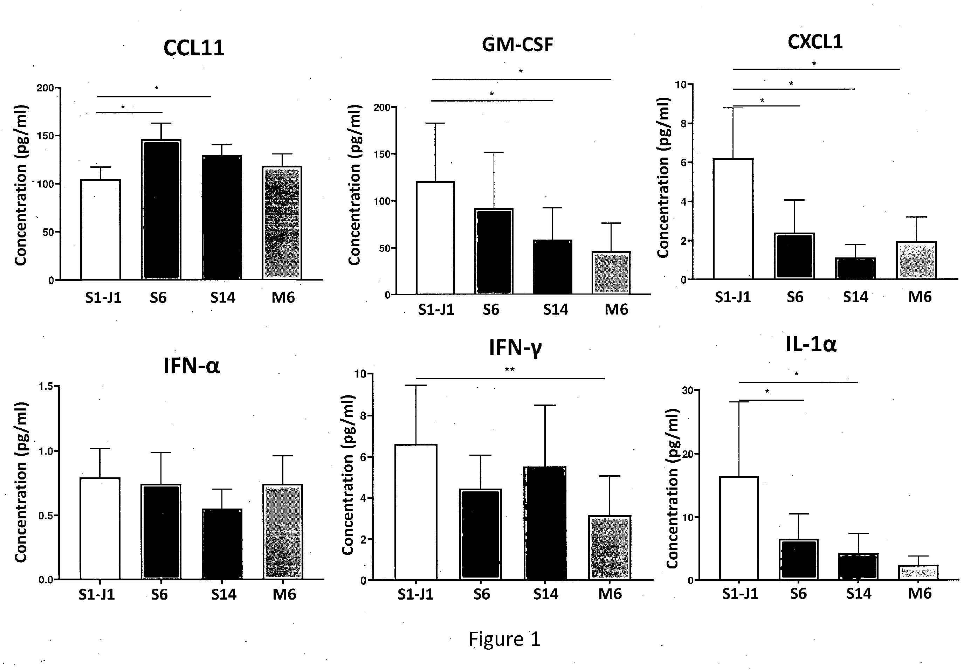

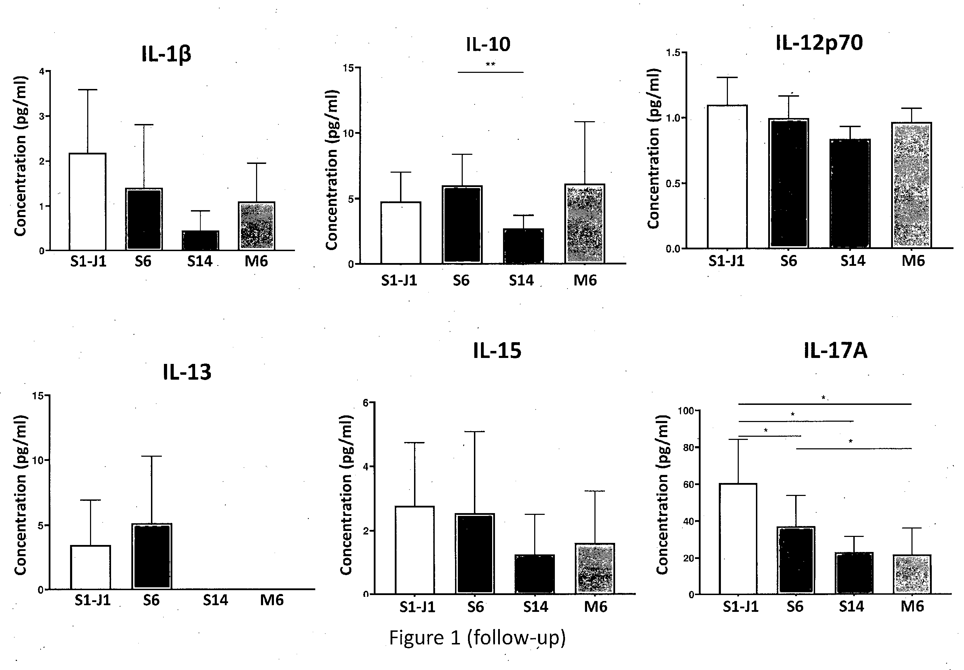

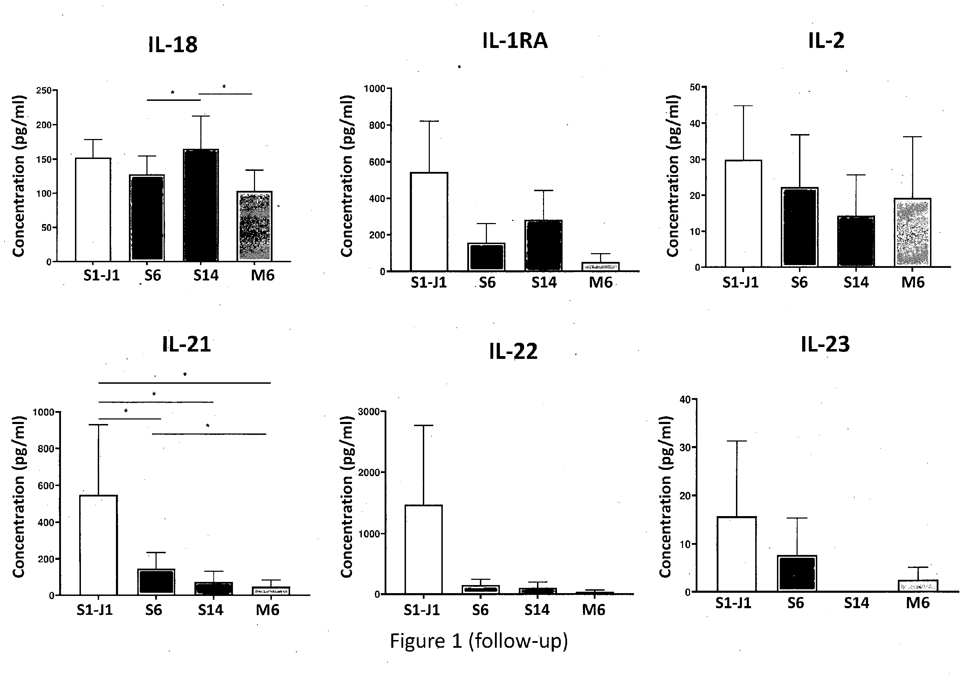

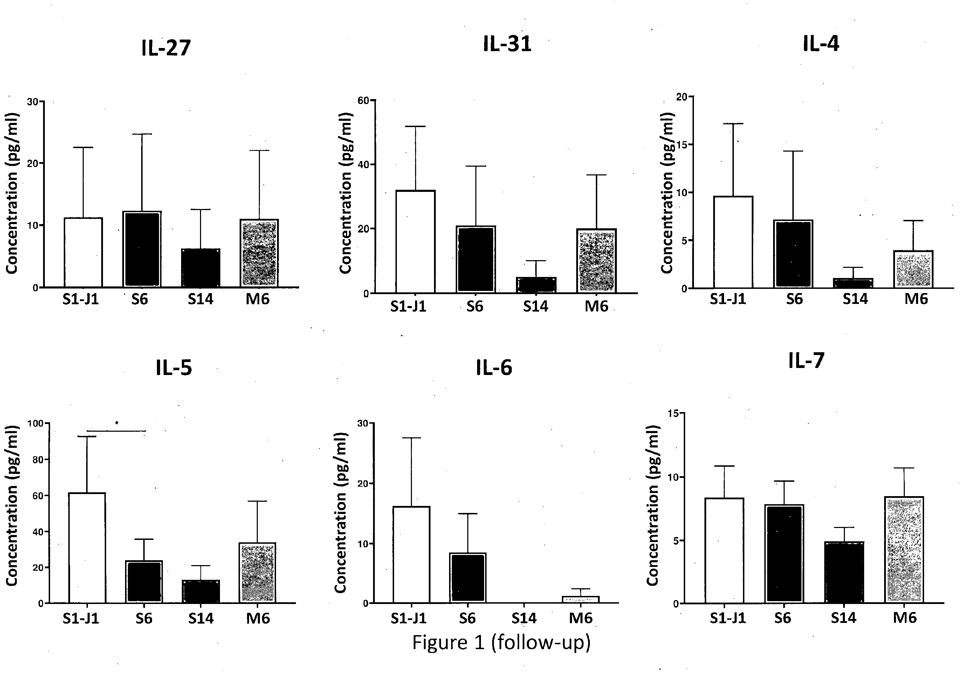

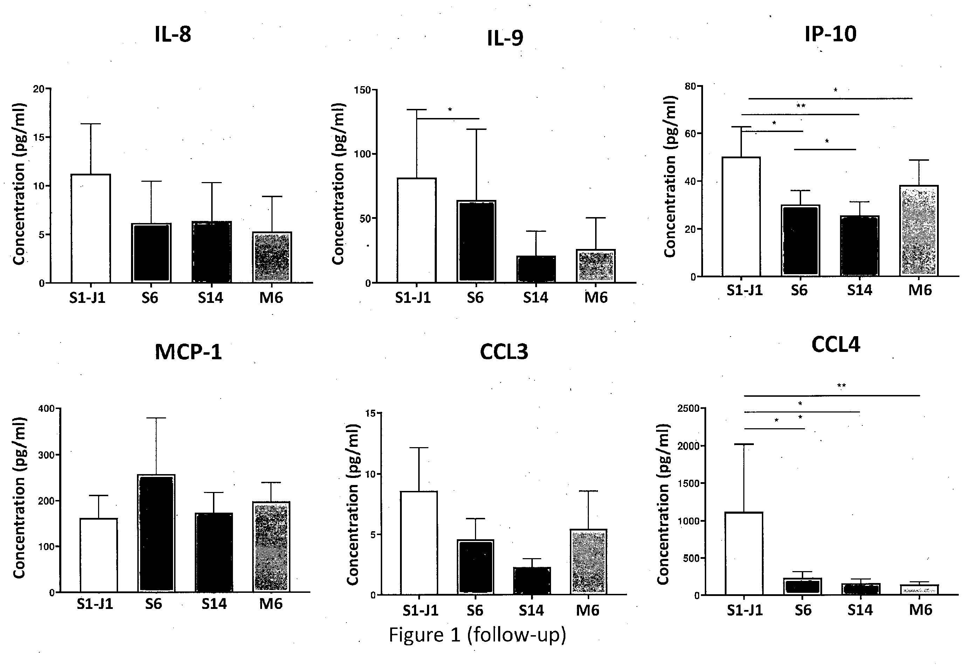

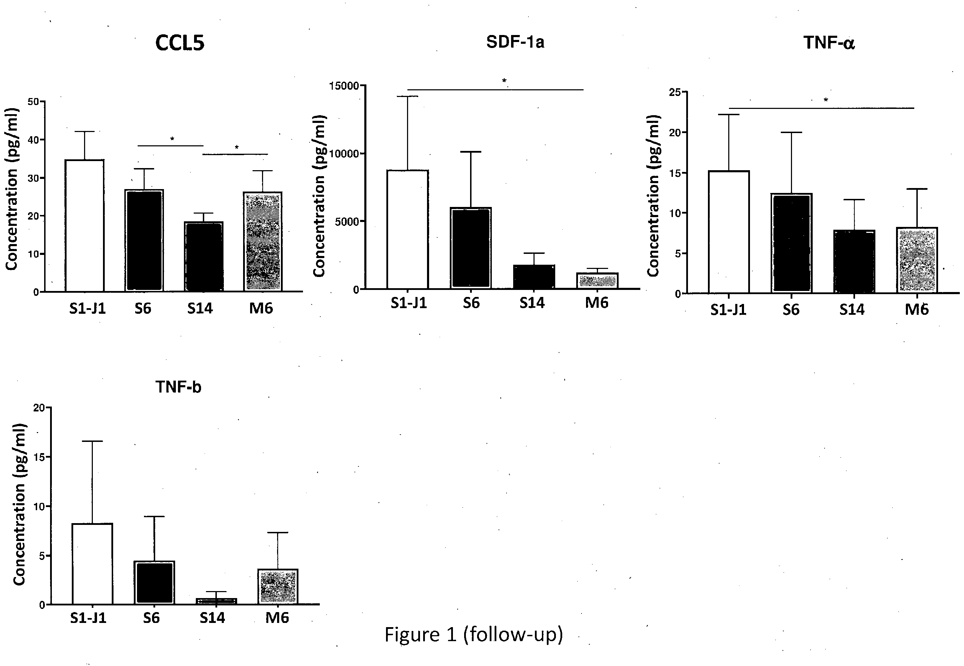

**Figure 1****: Plasma levels of Cytokine & Chemokine a 34-Plex Human ProcartaPlex^{™} Panel 1A kit (Thermo Fisher, # EPX340-12167-901).** Patients (n=20) with complete (CR) or partial (PR) response at 6 months after first infusion of ATO are represented in these graphs. Blood samples were obtained at inclusion (S1-J1) before each treatment administration, S6, S14 and M6. Plasma and erythrocytes were frozen on-site. Error bars represent SEM. Statistical differences were calculated using GraphPad Prism 8.4.2. A Wilcoxon t-test was performed. *p<0.05; **p<0.01; ***p<0.001.

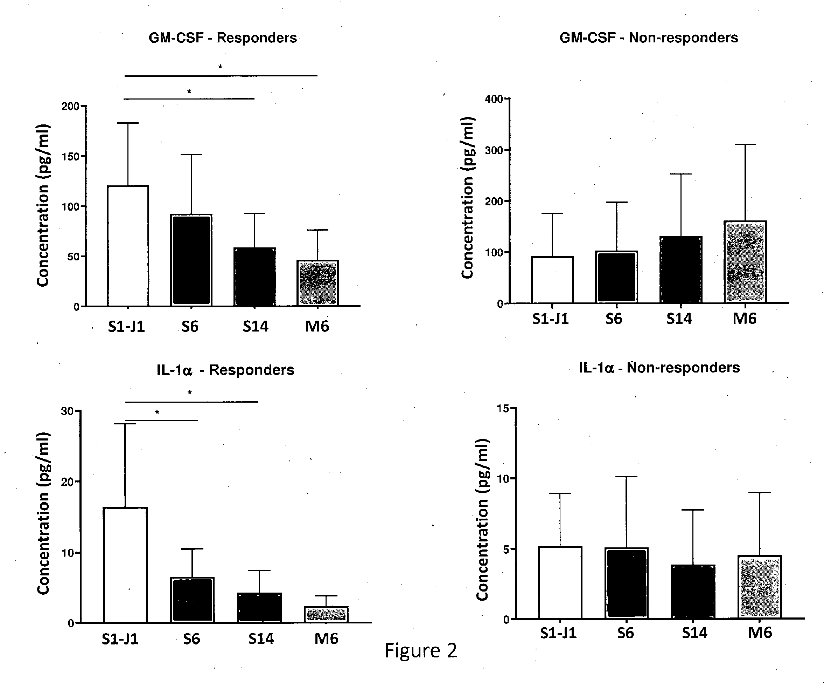

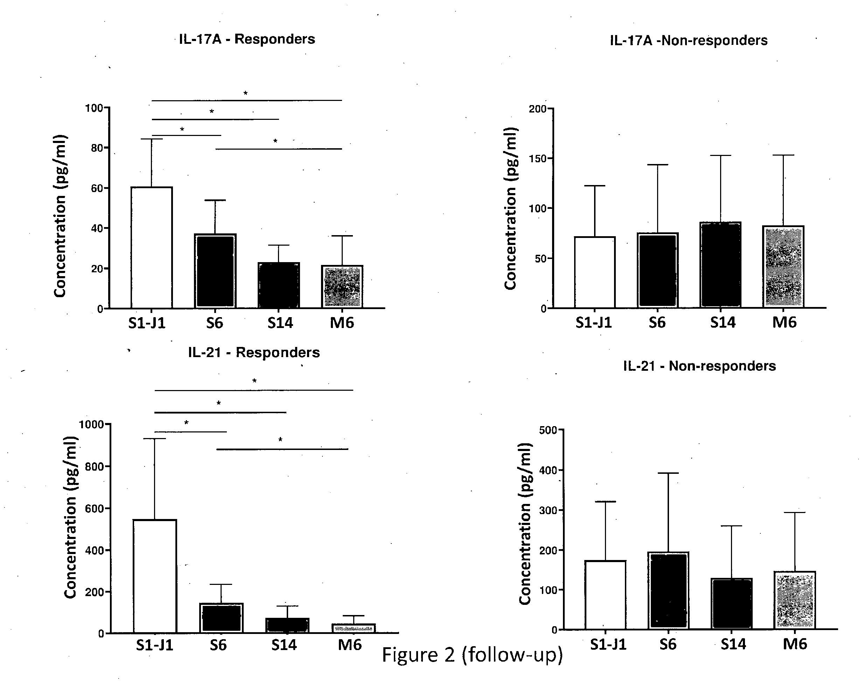

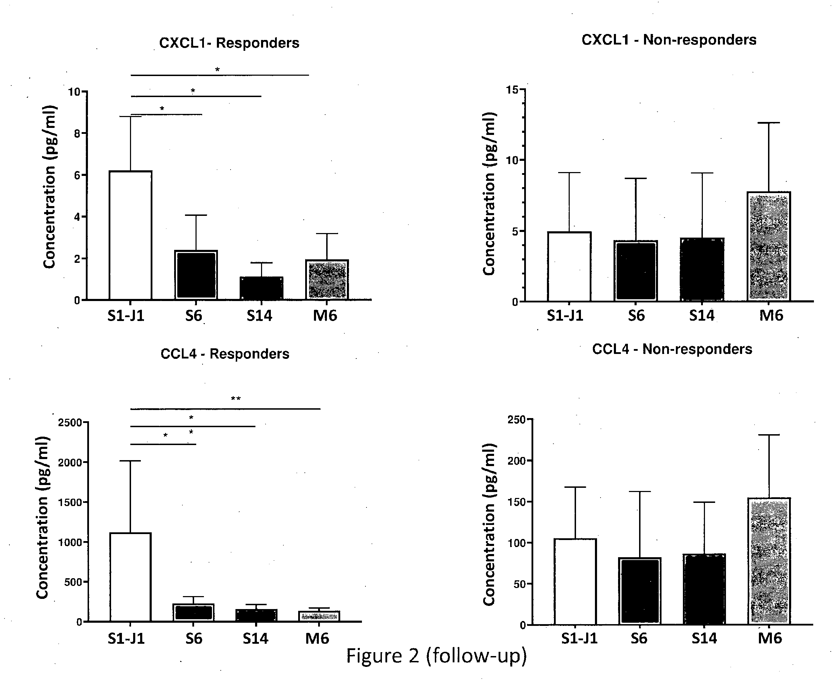

**Figure 2****: Cytokine & Chemokine plasma levels with different responses between responding and non-responding patients treated with ATO.** Patients with a complete (CR) or partial response (PR) are called Responders (n=15), and patients who did not respond are called Non-responders (n=5). Blood samples were obtained at inclusion (S1-J1) before each treatment administration, S6, S14 and M6. Plasma and erythrocytes were frozen on-site. Every concentration profile refers to the average concentration of each of the 34 cytokines in the 20 patients entering the efficacy analysis (FAS population). Error bars represent SEM. Statistical differences were calculated using GraphPad Prism 8.4.2. A Wilcoxon t-test was performed. *p<0.05; **p<0.01; ***p<0.001.

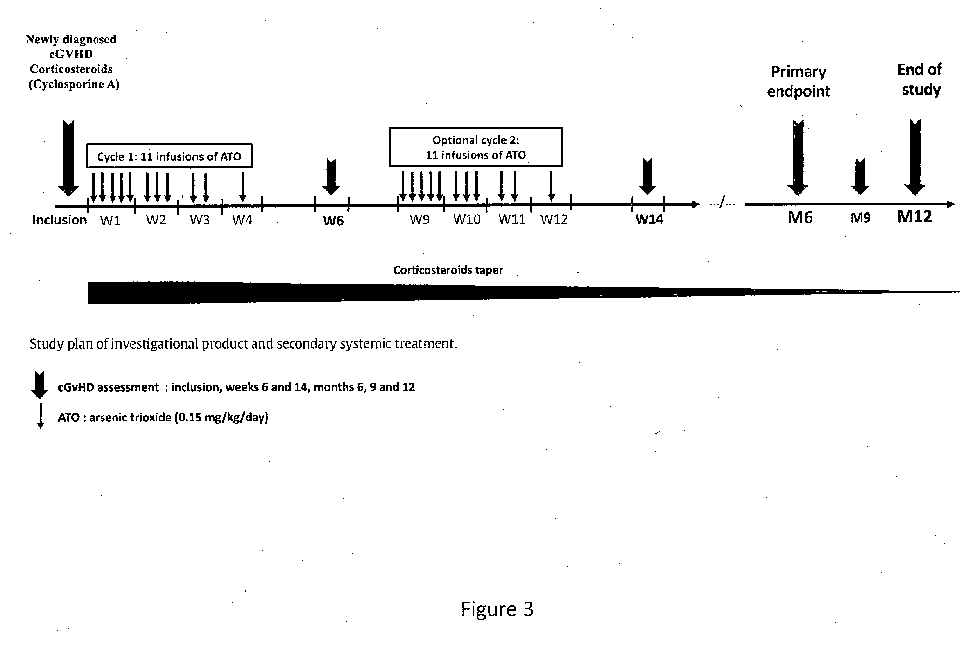

**Figure 3****:** Study plan of investigational product and secondary systemic treatment.

### DETAILED DESCRIPTION

As used herein, the words "comprise", or variations such as "comprises" or "comprising" as well as "including" and variations such as "include", "includes," and "included", are not limiting.

As used herein, the singular form "a", "an" and "the" include singular and plural references unless the context indicates otherwise.

The term "and/or", e.g., "X and/or Y" shall be understood to mean either "X and Y" or "X or Y" and shall be taken to provide explicit support for both meanings or for either meaning.

As used herein, the terms "level" or "level of a cytokine" or "level of the cytokines" refers to the concentration of GM-CSF and/or CCL4 and, optionally, of IL-1α, IL-17A, IL-21 and CXCL1, within a biological sample taken from an individual.

As used herein, graft-vs-host disease (GVHD) is defined as a disease in which lymphocytes and the like in a graft transplanted into a host of different histocompatibility antigen type, engraft in the host, and recognize host tissues as foreign and attack these tissues. In this case, the "host" is a recipient (transplant-benefiting patient) who has received blood infusion or cell transplantation.

As used herein, cGVHD is defined as the chronic form of GVHD. cGVHD generally manifests later than the acute form (aGVHD) (>100 days post transplantation). However, as already mentioned, during the development of cGVHD, there can be a period of time during which the acute and chronic forms of GVHD coexist. In this case, the acute and chronic forms are present at the same time in the same individual and independently of each other. cGVHD has many features of other, related autoimmune diseases. It may develop either de *novo,* following resolution of aGVHD or as an extension of aGVHD. cGVHD can cause multiple, often debilitating symptoms, including widespread skin rashes, painful mouth ulcers, shortness of breath, and limb and joint pain.

The method of the present Invention applies to the chronic form of graft-versus-host disease (cGVHD).

As used herein, the term "biomarker" refers to a biologic molecule that is endogenously produced and that can be objectively measured and used as an indicator of a normal biologic or pathogenic process. Furthermore, it can be used as an indicator of pharmacological response to a therapeutic intervention for reducing the disease caused by the pathologic process. In this case, the biomarker may be measured in sample(s) collected from the individual prior to and following treatment to monitor the course of the disease during treatment and support the clinician in taking appropriate clinical actions.

Other definitions will be specified below, when necessary.

According to a first aspect, the present Invention pertains to an *in vitro* method for the diagnosis, prognosis, stratification and/or monitoring of chronic graft-versus-host disease (cGVHD) in an individual who has received an allogeneic hematopoietic stem cell transplantation (allo-HSCT), comprising the steps of:
a) measuring the level of at least one cytokine selected from the group consisting of granulocyte-macrophage colony-stimulating factor (GM-CSF) and macrophage inflammatory protein (CCL4 or MIP-1β) in a biological sample from said individual,
b) optionally, measuring the level of at least one cytokine selected from the group consisting of Interleukin-1 alpha (IL-1α), Interleukin-17A (IL-17A), Interleukin-21 (IL-21) and chemokine (C-X-C motif) ligand 1 (CXCL1), in a biological sample from said individual,
c) comparing each of the levels measured in a) and, optionally, in b), to a reference value,
wherein levels in the biological sample higher than the reference values are indicative for the presence and/or aggravation of cGVHD.

As used herein, the term "diagnosis" preferably means determining the presence or the absence of cGVHD in a subject and/or determining whether a subject is at risk of developing cGVHD as well as predicting a status of cGVHD.

As used herein, the term "prognosis" refers to an estimate, based on medical experience and judgment of the health status of an individual, of the likely or expected development of cGVHD, including whether the signs and symptoms of cGVHD will improve or worsen (and how quickly) or remain stable over time.

As used here, the term "stratification" or "patient stratification" refers to the assignment of an individual diagnosed with cGVHD to a specific category of severity of the disease, e.g. to one of the categories established by the NIH guidelines (Jagasia *et al.* 2015).

As used herein, the term "monitoring" refers to the activity of following the status of an individual, where said individual might or might have not been diagnosed with cGVHD. In case the individual has not been diagnosed with cGVHD, the monitoring is intended to determine whether said individual will develop cGVHD over time. In case the individual has been diagnosed with cGVHD, the monitoring aims at evaluating the progression of the disease. In this case, the monitoring of disease progression in the individual can be performed during an ongoing treatment for cGVHD, but also before or after said individual has been administered any treatment for cGVHD.

As used herein, the terms "individual", "patient" and "subject" are used interchangeably. They refer to a mammal (e.g., a human) which is the object of a treatment, or observation.

The present Invention is mainly targeted at a human and is suitably used for human patients. However, the present Invention may be used for non-human animals in which at least antibody formation by immune reactions is observed.

The individual is a subject who has received a bone marrow or peripheral, hematopoietic stem cell transplantation as a consequence of a malignant or non-malignant hematologic disease.

The disclosed method for diagnosing cGVHD is independent of whether the subject has or has not been previously treated for cGVHD. Thus, the biological sample from the subject may be a sample from a subject who has been previously treated for cGVHD as well as a sample from a subject who has not been previously treated for cGVHD. In addition, the biological sample from the subject may be a sample from a subject who has already developed or who is developing acute GVHD as well as a sample from a subject who has never developed aGVHD. Also, the method for diagnosing cGVHD in a subject is independent from the progression status of cGVHD in the subject. More specifically, the method allows for diagnosing cGVHD in a subject having a mild as well as a moderate or severe form of cGVHD.

As used herein, the term "measuring" refers to methods which include detecting the presence or absence of a cytokines in a biological sample and quantifying its concentration. Additional information on the techniques usable in the present method can be found hereinafter.

Hence, the method of the Invention requires that at least the level of GM-CSF or CCL4 be measured

According to a specific embodiment, in step a), the level of GM-CSF is measured. According to another specific embodiment, in step a), the level of CCL4 is measured. According to another specific embodiment, in step a), the levels of GM-CSF and CCL4 are measured.

According to another specific embodiment, the level of at least one cytokine selected from the group consisting of GM-CSF and CCL4 is measured, alone or combined with the level of one, two, three or four of the cytokines selected from the group consisting of IL-1α, IL-17A, IL-21 and CXCL1.

The inventors have found that the level of the following cytokines correlated with the presence of cGVHD and its progression:
- GM-CSF, also known as Colony-stimulating factor, CSF, colony stimulating factor 2, CSF2, Granulocyte-macrophage colony-stimulating factor, Molgramostin and Sargramostim. GM-CSF could drive GVHD pathology by allowing donor-derived phagocytes to produce inflammatory mediators such as interleukin-1β and reactive oxygen species (Tugues S *et al.* 2018). Despite its original designation as a hematopoietic growth factor, mice lacking GM-CSF or its receptor develop normally and have a functional myeloid cell compartment, but exhibit specific defects in pulmonary immuno-physiology (Stanley E *et al.* 1994) and in the homeostasis of dendritic cells (DCs) in non-lymphoid tissues (Greter M *et al*. 2012). These past observations suggest that an increase in GM-CSF may be accompanied by various possible detrimental consequences on an otherwise normal immune system. Indeed, GM-CSF produced by activated T helper cells has a profound impact on the differentiation and activation of several myeloid cell subsets during pathologic tissue inflammation (Becher B *et al.* 2016; Codarri *L et al.* 2011; Croxford AL *et al*. 2015) and can overdrive myeloid cell cytokine production (Fleetwood AJ *et al.* 2007), phagocytosis (Croxford AL *et al.* 2015) and oxidative burst (LeVine AM *et al.* 1999). GM-CSF was first described in the conditioned media of mouse lung tissue following LPS injection, as a growth factor that induces the differentiation and proliferation of myeloid progenitors in the bone marrow (Shiomi A *et al.* 2015). GM-CSF is produced by multiple cell types such as activated T cells, B cells, macrophages, monocytes, mast cells, vascular endothelial cells and fibroblasts (Becher B *et al.* 2016). GM-CSF also has an important cellular effect in chronic inflammatory diseases by stimulating the activation and migration of myeloid cells to inflammation sites, promoting survival of target cells and stimulating the renewal of effector granulocytes and macrophages. Because of these cellular effects, an increase in GM-CSF production/signaling may lead to harmful inflammatory conditions in various organs. In this context, GM-CSF may have a prominent pathogenic role in other related autoimmune diseases that are dependent on cellular immune responses such as inflammatory bowel diseases, allergic disease, osteoarthritis, multiple sclerosis, rheumatoid arthritis and the like (Lee KMC *et al.* 2020);
- CCL4, which is a small cytokine that belongs to the CC chemokine subfamily. CCL4 is being secreted under mitogenic signals and antigens and acts as a chemoattractant for natural killer cells, monocytes and various other immune cells in the site of inflamed or damaged tissue. CCL4 contributes to the accumulation of alloreactive T-cells in GVHD;

- Interleukine 1alpha (IL-1α), which is an initiator of several major human diseases. Certain unique properties of IL-1α support its critical function as an apical instigator of inflammation. Polymorphisms of the IL-1α gene has been implicated in a number of autoimmune or inflammatory conditions;
- Interleukines 17A and IL-17A, which are implicated in immune responses to infectious pathogens and in the pathogenesis of inflammatory autoimmune diseases like psoriasis. The skin and serum IL-17 levels positively correlate with cGVHD severity (Yoshizaki A *et al.* 2010; Murata M *et al.* 2008; Resende RG *et al.* 2014; Flynn R *et al.* 2016);
- Interleukine 21 (IL-21), which has potent regulatory effects on cells of the immune system, including natural killer (NK) cells and cytotoxic T cells that can destroy virally infected or cancerous cells. IL-21 induces cell division/proliferation in its target cells. IL-21 may contribute to the development of cGVHD (Flynn R *et al.* 2016; Zhao X *et al.* 2013; MacDonald KPA *et al.* 2017);
- CXCL1, which is a small peptide that acts as a chemoattractant for several immune cells. It contributes to neutrophil activation during inflammation, especially neutrophils or other non-hematopoietic cells to the site of injury.

It is an embodiment of the present Invention that the levels of the biomarkers measured at the different time points, wherein different time points means at least two time points, may be compared. Without wishing to be bound by any theory the present inventors have found that the level of the biomarkers of the present Invention in biological samples from an individual can be correlated to the diagnosis of cGVHD in said individual at the time point the sample from the patient is taken.

It will be thus immediately understood by the skilled person that an elevated level of the biomarkers determined in the sample of a later time point compared to the level of the biomarkers determined in the sample taken at an earlier time point is indicative for a more severe status of the individual at the later time point compared to the status of the individual at the earlier time point.

On the contrary, a decreased level of the biomarkers determined in the sample of a later time point compared to the level of the biomarkers determined in the sample of an earlier time point is indicative for a less severe status of cGVHD in the individual at the later time point compared to the status of cGVHD in the individual at the earlier time point.

Accordingly, the present Invention provides a method for determining the time course and severity of cGVHD in an individual, wherein the method comprises the step of determining the level of one or more biomarker(s) present in biological samples taken from the individual at different time points. As a consequence, the method of the present Invention allows for a dynamic measurement (*i*.*e*., a kinetic profile) of the level of one or more of the cytokine(s) described herein.

For each time point at which a biological sample is collected, the measurement of the level of one or more of said cytokine(s) can refer to a single measurement or it can represent the average of several measurements (e.g., three measurements).

By allowing a dynamical measurement, the method of the present Invention allows for the diagnosis of cGVHD in an individual who has received an allo-HSCT and which is therefore at risk of developing the cGVHD after transplantation. This is of importance given the fact that, as explained above, the diagnosis of cGVHD by current clinical means is inadequate.

Given the importance, in many real case scenarios, to diagnose cGVHD as fast as possible, there is a requirement that diagnostic methods can be carried out quickly, in order to provide medical practitioners with reliable information obtained in as quickly a manner as possible so that they can take timely medical decisions. In addition, there is also a requirement on the sensitivity and specificity of the diagnostic methods. By allowing the diagnosis of cGVHD with high sensitivity, high specificity and in a timely manner, the method of the present Invention represents a clear improvement over the performance of the biomarkers and methods described in the art. Other advantages of the present method with respect to the art reside in its simplicity, reliability and cost-effectiveness.

As used herein, the term "higher than" is to be intended as "significantly higher than" and indicates that an increase of 5%, preferably of 10%, even more preferably of 15% is to be considered as statistically significant. At the same time, the term "lower than" is to be intended as "significantly lower than" and indicates that a decrease of 5%, preferably of 10%, even more preferably of 15% is to be considered as statistically significant. The terms "higher than" and "lower than" are based on the notion of "statistical significance" which, as used herein, refers to the measurement of the level of one or more of the cytokines of the Invention with respect to the reference value of said one or more cytokines.

In particular, the term "statistical significance" indicates whether the observed increase or decrease in the level of one or more of the cytokines with respect to the reference value is the result of chance or is attributable to a specific cause.

As used herein, "aggravation of cGVHD" refers to any worsening of severity, to any increase in the symptoms or scoring system or to the acceleration of cGVHD progression.

Based on the dynamical measurement of one or more of the biomarkers described herein, the method of the present Invention also allows for the prognosis, *i.e.* for the prediction of the likely or expected development of cGVHD. This is of importance given that the prognosis of cGVHD by current clinical means is inadequate.

The method of the present Invention may also be used for the stratification of the severity of cGVHD in a subject (*i.e.*, as mild, moderate or severe cGVHD, according to the NIH guidelines), which is important in the clinical practice in order to identify those patients that suffer from the severe form(s) of cGVHD, which in turn will allow an appropriate follow-up and individualized treatment. This could be of great impact because it could potentially avoid or reduce one or more complications typical of severe cGVHD.

Another advantage of the method of the present Invention is that it allows for monitoring the effectiveness of a certain treatment in a subject diagnosed with cGVHD.

In fact, according to the method of the present Invention, after the levels of one or more of the cytokine(s) described herein have been measured, each of said levels are compared to a reference value. The level of each cytokine is compared to the reference value for the same cytokine. By comparing the measured levels of the biomarkers in the sample from the subject, the skilled physician will recognize whether the level of the biomarkers increase, decrease or whether a stable level of the biomarkers is maintained over time. Levels in the biological sample higher than the reference value are indicative for the presence and/or aggravation of cGVHD. On the contrary, levels in the biological sample lower than the reference value are indicative of remission or absence of cGVHD. It will be immediately understood that, based on the result of such comparison, the skilled physician may decide to select and initiate a therapy and/or adjust the doses and/or dosage of a selected therapy. When adjusting the doses and/or dosage of a selected therapy, the skilled physician may decide whether to increase, reduce or maintain constant the dosage of the therapy based on the results of the comparison.

For the calculation of the temporal concentration profiles as well as for the comparison between levels of the biomarkers at different time points to reference values, methods of statistical analysis can be used. The statistical analysis may be performed by methods appropriately selected from various statistical methods and test methods known in the art. Preferably, a statistical analysis method suited for analysing temporal changes in discrete data is used, and the significance of the statistical results and the degree of risk are presented. The skilled person knows the methods of statistical analysis described in the art and how to present the results of such analysis in a meaningful way.

As used herein, the term "reference value" refers to the value that is used for the comparison against a level of one or more of the cytokines of the Invention measured at a certain time point in an individual and which allows establishing, after such comparison, the status of cGVHD in said individual. The reference value can be (i) an 'internal' value taken from a previous sample from the same individual, (ii) an 'internal' value measured from a previous sample from the same individual prior to allo-HSCT, (iii) an 'internal' value measured from a previous sample from the same individual before or after the onset of cGVHD, or it can be (iv) an 'external' value measured from a cohort of healthy individuals.

Therefore, according to the present Invention, the reference value can be an 'internal' or 'external' value, depending whether it has been obtained from the same individual or from a different individual or group of individuals (e.g., a study cohort), respectively; and it can be measured from a biological sample either before allo-HSCT or before or after the onset of cGVHD.

In the case the reference value is measured from an individual before the onset of cGVHD, the variation (i.e. an increase) of the level of one or more of the cytokines of the Invention will indicate whether said individual is developing the disease.

In the case the reference value is measured from an individual after the onset of cGVHD, the variation of the level of one or more of the cytokines of the Invention will indicate whether there is a worsening or a lessening of the disease.

As used herein, the term "cohort" refers to a group of individuals who share a defining characteristic, which in this case corresponds to the absence, in the individuals being part of the cohort, of any past or current treatment likely to cause cGVHD, e.g. an allo-HSCT. The skilled person knows how to determine the appropriate size of the cohort so as to guarantee the statistical significance of the data obtained studying such cohort. For example, the cohort implemented in the present Invention may comprise at least 3 individuals.

According to a particular embodiment of the Invention, the reference value for each cytokine is a level of said cytokine measured in a previous sample from the individual.

According to another particular embodiment of the Invention, the previous sample from the individual has been taken prior to the allo-HSCT, and a level of one or more of said cytokine(s) in the biological sample taken after the allo-HSCT higher than the reference value(s) indicates that the individual is likely to have or develop cGVHD.

According to this aspect, the reference value is the level of one or more of the cytokines of the Invention measured before the allo-HSCT.

According to another particular embodiment of the Invention, the reference value for each cytokine is a level of said cytokine measured in a cohort of healthy volunteers, and wherein a level of one or more of said cytokine(s) in the biological sample higher than the reference value indicates that the individual is likely to have or develop cGVHD.

As used herein, a subject is considered to be a healthy volunteer with regard to cGVHD, if the subject has not received any treatment, e.g. an allo-HSCT, likely to cause cGVHD.

According to this aspect of the Invention, the reference value for each cytokine is a level of said cytokine measured in a cohort of healthy volunteers.

The skilled person knows how to select healthy individuals to be part of the cohort, *i.e.* the skilled person knows which inclusion criteria or characteristics (e.g., health status, age, sex, geographical origin *etc*...) the individuals need to fulfill to be part of the cohort.

According to another embodiment of the present Invention, the reference value for each cytokine is a level of said cytokine measured in a previous sample from the individual, taken after the onset of cGVHD, and a level of one or more of said cytokine(s) in the biological sample higher than the reference value is indicative for an aggravation of cGVHD.

As used here, the term "onset of cGVHD" refers to the first appearance of the signs or symptoms of cGVHD. The onset of cGVHD may be determined by using the method of the present Invention and/or by a clinical practitioner following the consensus criteria for the diagnosis of cGVHD described in the NIH Guidelines (Jagasia *et al.* 2015).

The reference value for each cytokine may also be the level of the one or more cytokines of the Invention measured in a previous sample from the individual, and taken before the onset of cGVHD. In this case, the variation of the level of one or more of the cytokines of the Invention will indicate whether said individual is developing the disease.

According to another embodiment, the method of the present Invention is used to evaluate if an individual who has received a treatment for cGVHD is responsive to said treatment. In a particular embodiment, a level of one or more of the cytokine(s) of the Invention in the biological sample taken from the individual under treatment for cGvHD lower than the reference value is indicative for a response to the treatment.

According to the present Invention, the treatment received by the individual can be any treatment known in the art to be useful for treating cGVHD.

This treatment may be an immunosuppressive treatment or an immunomodulatory treatment.

As already mentioned, prednisone is the standard first-line therapy for cGVHD. For patients who do not respond to steroid treatment, there are second-line treatments. Ibrutinib (Imbruvica) is an FDA-approved drug for the treatment of adult patients with cGVHD after failure of one or more lines of systemic therapy. Other treatments include, but are not limited to, the administration of mycophenolate mofetil, sirolimus, tacrolimus or cyclosporine, monoclonal antibodies such as infliximab (Remicade), tocilizumab (Actemra), alemtuzumab (Campath), basiliximab (Simulect), daclizumab (Zinbryta) and denileukin diftitox (Ontak), antithymocyte globulin (ATG), pentostatin (Nipent) and ruxolitinib (Jakafi). ATO is also used for the treatment of cGVHD. In addition, extracorporeal photopheresis (ECP) is being developed for the treatment of cGVHD.

As used herein, "response to treatment" refers to any lessening of severity, delay in onset, slowing of progression, or shortening of duration of cGVHD, whether permanent or temporary, lasting or transient.

In the methods according to any of the preceding aspects, the biological sample can be selected from the group consisting of blood such as whole blood or anti-coagulated whole blood, blood serum, blood plasma, lymph, saliva, cerebrospinal fluid, interstitial fluid, an isolated bone marrow fluid, respiratory tract fluid, cellular extracts, tissue extracts and organ extracts.

Thus, the biological sample can be any fluid naturally secreted or excreted from a human or animal body or any fluid recovered, from a human or animal body, by any technique known to the skilled person such as extraction, taking, recovering or washing. These steps of recovery or isolation of the biological sample from the human or animal body are carried out prior to the implementation of the process according to the Invention.

The skilled person knows how to collect and store the biological sample so as to avoid possible degradation of the biomarkers described herein.

For example, if the biological sample is to be tested immediately, e.g., when it is suspected that the patient is developing cGVHD, the sample can be maintained at room temperature, preferably for no longer than 6 hours; otherwise, the sample can be refrigerated or frozen (e.g., at -80°C) prior to the assay.

In particular, the biological sample implemented in the present Invention is a sample of blood, plasma or serum, preferably a serum sample.

According to another embodiment of the present Invention, the level of the one or more cytokine(s) described herein is measured by immunoassay.

According to this preferred aspect of the Invention, the concentration of one or more of the biomarkers described herein can be determined at the mRNA and/or at the protein level and/or by Mass Spectrometry (MS), High Performance Liquid Chromatography (HPLC) or HPLC coupled to MS (e.g., HPLC-MS/MS), without this limiting the Invention.

Immunoassays for the determination of the concentration of the biomarkers at the mRNA level include, but are not limited to, northern blot, in situ hybridization, techniques based on the use of reverse transcriptase, especially in combination with (PCR), such as quantitative PCR, quantitative real time (RT)-PCR, or any other method for RNA analysis.

Immunoassays for the determination of the concentration of the biomarkers at the protein level include, but are not limited to, RIA (Radioimmunoassay), Competitive EIA (Competitive Enzyme Immunoassay), DAS-ELISA (Double Antibody Sandwich-ELISA), immunocytochemical techniques, immunohistochemical techniques, techniques based on the use of biochips, techniques based on the use of protein microarrays that include specific antibodies, assays based on the precipitation of colloidal gold in formats, affinity chromatography techniques and ligand binding assays, or any other method of protein analysis.

Immunoassays can be conducted using any format known in the art, such as, but not limited to, a sandwich format.

The present Invention also claims a method for determining if an individual responds to a treatment of cGVHD, comprising measuring the level of one or more of the cytokine(s) described herein and according to any of the embodiments above, wherein a decrease in the level of one or more of said cytokines indicates that the individual responds to the treatment.

In other words, the present Invention concerns a method for determining if an individual responds to a treatment of cGVHD, comprising performing the detection, prognosis, stratification and monitoring method as previously defined at different time points, wherein a decrease in the level of one or more of said cytokines indicates that the individual responds to the treatment.

According to this aspect of the Invention, the present method is a theranostic method, *i.e.* it combines diagnosis and specific targeted therapy. Hence, another advantage of the method of the present Invention is that it allows achieving a personalized treatment approach to the patient. Being based on biomarker profiling, the theranostic method of the present Invention allows tailoring optimized therapies based on the levels of biomarkers of an individual, thereby reducing side-effects and improving response to treatment.

The measurement of the level of one or more cytokine(s) described herein at different time points in a biological sample of an individual can be performed at different moments, e.g., before allo-HSCT, immediately after allo-HSCT, at the onset of the symptoms evocative of cGVHD, at the onset of the cGVHD treatment, preferably at the onset of cGVHD treatment.

As used herein, the meaning of expressions such as "decrease" and "significant decrease" is to be intended as equivalent to that of the terms "lower than" and "significantly lower than" which have been explained above.

The present Invention also concerns the use of GM-CSF and/or CCL4, alone or combined to one, two, three or four cytokines selected from the group consisting of IL-1α, IL-17A, IL-21 and CXCL1, as a biomarker for the diagnosis, prognosis, stratification and/or monitoring of cGVHD in a subject who has received an allo-HSCT.

The present Invention also concerns a method of treating cGVHD in an individual in need thereof, comprising the steps of:
a) measuring the levels of the cytokines as previously defined in biological samples taken from the individual at different time points;
b) administering a medication for treating cGVHD such as corticosteroid (CS) and/or ATO to the individual; and
c) adapting the dosage regimen of said medication(s) for treating cGVHD such as CS and/or ATO depending on the evolution of the levels of said cytokines, wherein the dosage is tapered down if the levels decrease and can be tapered off if the levels become inferior to normal reference values.

As used herein, the terms "treat", "treatment" or "treating" include lessening of severity of cGVHD, delay in onset of cGVHD, causing regression of cGVHD, relieving a condition caused by cGVHD, or stopping symptoms which result from cGVHD. The terms "treat," "treating" or "treatment", include, but are not limited to, prophylactic and/or therapeutic treatments. In addition, the terms "treat," "treating" or "treatment" refer to any current or future treatment for cGVHD.

According to the present Invention, the medication to be administrated at step b) of this aspect of the invention can be any treatment known in the art for treating cGVHD and previously disclosed.

Indeed, the one or more cytokine(s) according to the present Invention are biomarkers of cGVHD, regardless of the specific treatment administered to the individual. In other terms, it is reasonable to expect that, for any given treatment capable of alleviating cGVHD, there is a concomitant decrease in the concentration of said one or more cytokine(s).

For example, and as already mentioned, prednisone is the standard first-line therapy for cGVHD. Any type of immunosuppressive or immunomodulatory treatments can be used. For patients who do not respond to steroid treatment, there are second-line treatments. Ibrutinib (Imbruvica) is an FDA-approved drug for the treatment of adult patients with cGVHD after failure of one or more lines of systemic therapy. Other treatments include, but are not limited to, the administration of mycophenolate mofetil, sirolimus, tacrolimus or cyclosporine, monoclonal antibodies such as infliximab (Remicade), tocilizumab (Actemra), alemtuzumab (Campath), basiliximab (Simulect), daclizumab (Zinbryta) and denileukin diftitox (Ontak), antithymocyte globulin (ATG), pentostatin (Nipent) and ruxolitinib (Jakafi). ATO is also used for the treatment of cGVHD. In addition, third-line therapies, e.g., extracorporeal photopheresis (ECP), are also being developed for the treatment of cGVHD. Other medications, already used or to be adopted in the clinical setting, can also be considered for treating cGVHD.

In particular, the medication to be administrated at step b) of this aspect of the invention can be corticosteroid and/or ATO. The corticosteroid to be used for this medication can be any corticosteroid, e.g. prednisone, prednisolone, methylprednisolone or mixture thereof.

Hence, according to the method of the Invention, any corticosteroid can be administered alone and/or in combination with other medicaments such as ATO.

According to the clinical trial performed by the inventors and described in Rongvaux-Gaida *et al.* 2022, the dosage of ATO is kept constant over the course of the treatment, while the dosage of CS can be tapered down or tapered off if the levels of one or more cytokine(s) according to the present Invention decrease. Therefore, the clinician can modify the treatment and, in particular, the dosage of CS, after receiving information regarding the levels of one or more cytokine(s) described herein and measured over the course of the therapy. As it is known in the art, the prolonged use of CS is associated with important side effects and there is a need of new agents with a better safety profile and higher efficacy. Hence, one of the advantages of the method of the present Invention consists in the possibility to reduce the dosage of CS in the treatment of cGVHD.

As a consequence, the adaptation of the dosage regimen at step c) of the method according to the Invention may consist in adapting the dosage regimen of CS, in particular, when the medication at step b) is CS and ATO.

The normal reference value can be the reference value measured from a biological sample taken from the same individual before the beginning of the therapeutic treatment.

According to another aspect, the present invention also encompasses the use of a kit for the diagnosis, prognosis, stratification and/or monitoring of cGVHD, comprising reagents for measuring the level(s) of GM-CSF and/or CCL4 in a biological sample.

The kit allows the concentration of GM-CSF, CCL4, IL-1α, IL-17A, IL-21 and CXCL1 in blood samples from an individual affected by cGVHD to be measured, e.g., in triplicate. Preferably, the kit is used to measure the level of the cytokine(s) before and after any therapeutic treatment or before and after allo-HSCT.

According to a specific embodiment, the kit further comprises reagents for measuring the level(s) of one or several cytokines selected from the group consisting of IL-1α, IL-17A, IL-21 and CXCL1.

According to another specific embodiment, the kit may also comprise antibodies specifically recognizing the recited cytokine(s).

The antibodies employed in the diagnostic kit of the present Invention can be obtained using a variety of different techniques known in the art.

The antibodies (Abs) specifically recognizing the cytokine(s) of the Invention can be, but are not limited to,' monoclonal antibodies (mAbs), polyclonal antibodies, chimeric antibodies, recombinant fragments of an antibody, Fab fragments, Fab' fragments, F(ab')2 fragments, F fragments or single-chain variable fragments (scFvs).

According to still another embodiment, the kit comprises antibodies that are bound to a solid support.

According to this aspect, the antibodies can be immobilized to beaded agarose resin and other solid supports.

According to still another embodiment, the kit further comprises a biotinylated detection Ab.

The kit according to the present Invention can optionally include other reagents required to conduct a diagnostic assay or facilitate quality control evaluations, such as buffers, salts, enzymes, enzyme co-factors, substrates, detection reagents, and the like. Other components, such as buffers and solutions for the isolation and/or treatment of a sample, may also be included in the kit. The kit may additionally include one or more other controls. The various components of the kit optionally are provided in suitable containers. The kit can further include containers for holding or storing a sample. Where appropriate, the kit may also optionally contain reaction vessels, mixing vessels and other components that facilitate the preparation of reagents or the sample. The kit may also include one or more instruments for assisting with obtaining a sample, such as a syringe, pipette, forceps, measured spoon, or the like.

Other characteristics of the Invention will also become apparent in the course of the description which follows of the biological assays which have been performed in the framework of the Invention and which provide it with the required experimental support, without limiting its scope.

### EXAMPLES

### Materials & Methods

The experimental results shown in Example 1 have been obtained using the materials and methods described in Rongvaux-Gaida *et al.* 2022.

The experimental results shown in Example 2 have been obtained using the following materials and methods:

### Preparation of blood samples

In the method of the Invention, the specimen is plasma or serum prepared from individual patient's blood, rapidly frozen after collection (-80°C in a 30 min lapse time). The specimens have been collected from the patients involved in the clinical trial described in Example 1, *i*.*e*. from adult patients with cGVHD requiring systemic therapy after first allo-HSCT for a hematological disease.

According to the method of the Invention, the blood may be collected from any parts of the body without limitation. The blood may be collected in an amount sufficient to obtain enough serum for performing the ELISA assay. The blood amount is preferably between 1 to 2ml. The timing and number of blood collection may be referred to doctors for decision, but the blood is preferably collected at day 0 (before any therapeutic treatment), week 6, week 14, month 6, month 9 and month 12.

### Quantification of the 34 cytokines and chemokines

The concentration of the 34 selected cytokines and chemokines was measured by immunoassays based on the principles of sandwich ELISA. The immunoassays were performed on the cohort blood samples collected at different time points as described above with a Luminex instrument and using highly specific antibodies binding to different epitopes of the tested cytokines and chemokines using the kit Thermo Fisher # EPX340-12167-901.

### Statistical analysis of the Luminex data

Statistical differences were calculated using GraphPad Prism 8.4.2. A Wilcoxon t-test was performed.

Before any statistical analysis, 4 patients presenting uncommon cytokines (IL-13, IL-23, IL-27, TNF-β) profiles expression were removed because they were a strong bias in the analysis of their results.

In addition, the temporal concentration profiles for each of the 34 cytokines and chemokines were evaluated by considering the whole patients' cohort as well as by separating the patients' cohort in responders (R) and non-responders (NR).

The analytical sensitivity of the method for the detection of GM-CSF as put in practice by the inventors, *i.e.* by using the specific sandwich ELISA kit and the other experimental details given above, is < 3 pg/mL, the assay range is 7.8-500 pg/mL and the volume for one measurement is 50 µl of plasma or 50µl of serum.

The analytical sensitivity of the method for the detection of CCL4 as put in practice by the inventors, *i.e.* by using the specific sandwich ELISA kit and the other experimental details given above, is 2.5 pg/mL, the assay range is 4.5-1000 pg/mL and the volume for one measurement is 100 µl of plasma or 100µl of serum.

The analytical sensitivity of the method for the detection of IL-1α as put in practice by the inventors, *i.e.* by using the specific sandwich ELISA kit and the other experimental details given above, is 0.5 pg/mL, the assay range is 0.5-300 pg/mL and the volume for one measurement is 100 µl of plasma or 100µl of serum.

The analytical sensitivity of the method for the detection of IL-17A as put in practice by the inventors, *i.e.* by using the specific sandwich ELISA kit and the other experimental details given above, is 0.2 pg/mL, the assay range is 2.5-75 pg/mL and the volume for one measurement is 100 µl of plasma or 100µl of serum.

The analytical sensitivity of the method for the detection of IL-21 as put in practice by the inventors, *i.e.* by using the specific sandwich ELISA kit and the other experimental details given above, is 110 pg/mL, the assay range is 125-8000 pg/mL and the volume for one measurement is 100 µl of plasma or 100µl of serum.

The analytical sensitivity of the method for the detection of CXCL1 as put in practice by the inventors, *i*.*e*. by using the specific sandwich ELISA kit and the other experimental details given above, is 2 pg/mL, the assay range is 1.5-1000 pg/mL and the volume for one measurement is 100 µl of plasma or 100µl of serum.

### Example 1: Clinical trial to evaluate the efficacy and safety of a first-line combination of ATO and CS in adult patients with cGVHD requiring systemic therapy after first allo-HSCT for a hematologic disease

In what follows, the clinical trial performed by the inventors to evaluate the efficacy and safety of a first-line combination of ATO and CS in adult patients with cGVHD is described. More details regarding the clinical trial (e.g., patient's inclusion and exclusion criteria, ATO products used for ATO administration, specific modality of administration of ATO and CS, *etc*...) can be found in the recent publication of the inventors (Rongvaux-Gaida *et al.* 2022).

As mentioned above, the inventors carried out a clinical trial with the aim to evaluate the efficacy and safety of a first-line combination of ATO and CS in adult patients with cGVHD requiring systemic therapy after first allo-HSCT for a hematologic disease. The study plan is reported Figure 3. In this clinical trial, ATO was started within 10 days of CS standard of care. Patients received 11 infusions per cycle of 24 days at a dose of 0.15 mg/kg per day. According to the clinical response and depending on the clinician's opinion, patients received a second cycle of treatment. Cycles were separated by an 8- to 11-week interval from the first infusion of ATO. Patients were evaluated and their blood was drawn at baseline (D0), 6 weeks, 14 weeks, 6 months, 9 months, and 12 months after the first ATO infusion. Additional blood samples were taken during each treatment cycle by the ATO (D0, D8 and D24). The primary endpoint of the study was preliminary efficacy based on the overall response rate (ORR; complete response [CR] or partial response [PR]) at 6 months.

The initial dose of CS could be tapered at 2 weeks after the first ATO infusion. A general guideline for CS weaning after 20 weeks was provided to the participating clinicians in the event of cGVHD clinical improvement (Table 1). It was suggested, but not required, that patients be tapered off steroids before tapering of other immunosuppressive medications.

21 patients entered the study and received at least 1 dose of ATO. 20 patients completed at least the first cycle of ATO treatment.

**Table 1: Prednisone taper schedule.**

| **Week** | **Dose (mg/kg actual body weight/day)** |
|---|---|
| 0 | 1.0 qod* |
| 2 | 0.75 qod |
| 4 | 0.65 qod |
| 6 | 0.50 qod (if CR, continued tapering of CS dose) |
| | 0.50 qod (if no CR, maintain this dose until resolution of all reversible clinical manifestations or adjust the required dose) |
| 8 | 0.40 qod |
| 10 | 0.30 qod |
| 12 | 0.20 qod |
| 14 | 0.15 qod |
| 20 | 0.0 qod |

| | |
|---|---|
| *qod: alternate daily dose (*i*.*e*., measured every other day, and calculated according to the patient's weight). | |

The clinical trial required a precise follow-up of the evolution of the disease. In particular, cGVHD was evaluated at each visit by the clinician using a dedicated form (Lee SJ *et al.* 2015). The primary efficacy variable was the response (complete or partial) at 6 months after first infusion of ATO. Complete response was defined as the complete disappearance of any sign of cGVHD (Lee SJ *et al.* 2015). Partial response was defined as an improvement of 1 point on a 4- to 7-point scale or an improvement of 2 points on a 10- to 12-point scale in at least 1 organ or site without progression in any other organ or site (Lee SJ *et al.* 2015). For patients with bronchiolitis obliterans syndrome, an absolute improvement in percentage of forced expiratory volume in 1 second of 10% predicted (e.g., 50% to 60%) was considered a PR as long as the initial value was < 70%. Normalization (80%) was considered a CR.

Clinical response was based on expert adjudication at 6 months for the main analysis and on NIH consensus criteria for the robustness assessment. Efficacy success was defined as CR or PR at 6 months after the first ATO infusion, with no secondary systemic therapy at any time. Treatment failure was defined as initiation of a new systemic treatment for cGVHD (other than CsA initiation in patients not receiving CsA at enrollment), recurrent or progressive malignancy, or death.

After the analysis of the data collected over the clinical study, the inventors observed that the first-line combination of ATO and CS was associated with a high clinical response rate and rapid CS sparing in cGVHD after previous allo-HSCT and concluded that the combination of ATO and prednisone was effective and well-tolerated in patients with cGVHD after allo-HSCT.

### Example 2: Determination of the temporal evolution of the concentration of a group of selected cytokines and chemokines in the sera of the patients enrolled in the clinical trial over the course of the treatment

After completion of the clinical trial described in Example 1, the inventors used the cohort sera collected over the course of the patients' treatment with the aim to measure the concentration profile of 34 cytokines and chemokines.

The levels of the 34 cytokines were measured in the sera of the 20 patients included in the efficacy analysis (FAS population).

The concentration profiles for the 34 cytokines and chemokines are shown in Figures 1 and 2.

Among the 34 selected cytokines and chemokines for which a temporal concentration profile was measured, six of them presented a remarkable and sharply modified profile over the time during the 1 year clinical trial follow-up. These are: GM-CSF, IL-1α, IL-17A, IL-21, CXCL1 and CCL4.

It is important to note that, for this sub-group, the temporal profile measured for the responder patients (R) show a decrease of the concentration of the cytokine or chemokine over the course of the treatment, while the profiles calculated for non-responder patients (NR)- show either a slight increase in the concentration or a rather stable profile over time.

The measurement of the concentrations of the 34 cytokines by sandwich ELISA technique in the patients enrolled in the clinical study, allowed to establish the significance of GM-CSF, CCL4, IL-1α, IL-17A, IL-21 and CXCL1 , as predictive biomarkers for cGVHD. The inventors demonstrated that the blood concentrations of GM-CSF, CCL4, IL-1α, IL-17A, IL-21 and CXCL1 are a cGVHD-specific measure capable of providing data useful in predicting the development of the disease.

The method established by the inventors can be advantageously put in the form of a diagnostic kit for the diagnosis, prognosis, stratification and/or monitoring of cGVHD.

### REFERENCES

Becher B, Tugues S, Greter M., GM-CSF: From Growth Factor to Central Mediator of Tissue Inflammation. Immunity. 2016; 45(5):963-73
Codarri L, Gyülvészi G, Tosevski V, Hesske L, Fontana A, Magnenat L, et al., RORγt drives production of the cytokine GM-CSF in helper T cells, which is essential for the effector phase of autoimmune neuroinflammation. Nat Immunol. 2011;12(6):560-7
Croxford AL, Spath S, Becher B. GM-CSF in Neuroinflammation: Licensing Myeloid Cells for Tissue Damage. Trends Immunol. 2015;36(10):651-62
Croxford AL, Lanzinger M, Hartmann FJ, Schreiner B, Mair F, Pelczar P, et al. The Cytokine GM-CSF Drives the Inflammatory Signature of CCR2+ Monocytes and Licenses Autoimmunity. Immunity. 2015;43(3):502-14
Dignan FL, Amrolia P, Clark A, et al. Diagnosis and management of chronic graft-versus-host disease. Br J Haematol. 2012;158:46-61
Fleetwood AJ, Lawrence T, Hamilton JA, Cook AD. Granulocyte-macrophage colony-stimulating factor (CSF) and macrophage CSF-dependent macrophage phenotypes display differences in cytokine profiles and transcription factor activities: implications for CSF blockade in inflammation. J Immunol Baltim Md 1950. 2007;178(8):5245-52.
Flynn R, Paz K, Du J, Reichenbach DK, Taylor PA, Panoskaltsis-Mortari A, et al. Targeted Rho-associated kinase 2 inhibition suppresses murine and human chronic GVHD through a Stat3-dependent mechanism. Blood 2016;127(17):2144-54
Fowler DH, Pavletic SZ. Syk and tired of current chronic GVHD therapies. Blood. 2015;125:3974-3975
Greter M, Helft J, Chow A, Hashimoto D, Mortha A, Agudo-Cantero J, et al. GM-CSF controls nonlymphoid tissue' dendritic cell homeostasis but is dispensable for the differentiation of inflammatory dendritic cells. Immunity. 2012;36(6):1031-46
Hart JW, Shiue LH, Shpall EJ, Alousi AM. Extracorporeal photopheresis in the treatment of graft-versus-host disease: evidence and opinion. Ther Adv Hematol. 2013;4:320-334
Jagasia MH, Greinix HT, Arora M, Williams KM, Wolff D, Cowen EW, et al. National Institutes of Health Consensus Development Project on Criteria for Clinical Trials in Chronic Graft-versus-Host Disease: I. The 2014 Diagnosis and Staging Working Group report. Biol Blood Marrow Transplant J Am Soc Blood Marrow Transplant. 2015;21(3):389-401.e1.
Lee KMC, Achuthan AA, Hamilton JA. GM-CSF: A Promising Target in Inflammation and Autoimmunity. ImmunoTargets Ther. 2020;9:225-40
Lee SJ, Wolff D, Kitko C, Koreth J, Inamoto Y, Jagasia M, et al. Measuring Therapeutic Response in Chronic Graft-versus-Host Disease. National Institutes of Health Consensus Development Project on Criteria for Clinical Trials in Chronic Graft-versus-Host Disease: IV. The 2014 Response Criteria Working Group Report. Biol Blood Marrow Transplant. 2015;21(6):984-99
Lee SJ, Onstad L, Chow EJ, Shaw BE, Jim HSL, Syrjala KL, et al. Patient-reported outcomes and health status associated with chronic graft-versus-host disease. Haematologica. 2018;103(9):1535-41
LeVine AM, Reed JA, Kurak KE, Cianciolo E, Whitsett JA. GM-CSF-deficient mice are susceptible to pulmonary group B streptococcal infection. J Clin Invest. 1999;103(4):563-9
MacDonald KPA, Blazar BR, Hill GR. Cytokine mediators of chronic graft-versus-host disease. J Clin Invest. 2017, 127(7):2452-63
Mahmoud HK, Elhaddad AM, Fahmy OA, Samra MA, Abdelfattah RM, El-Nahass YH, et al. Allogeneic hematopoietic stem cell transplantation for non-malignant hematological disorders. J Adv Res. 2015;6(3):449-58
Murata M, Fujimoto M, Matsushita T, Hamaguchi Y, Hasegawa M, Takehara K, et al. Clinical association of serum interleukin-17 levels in systemic sclerosis: is systemic sclerosis a Th17 disease? J Dermatol Sci. 2008;50(3):240-2
Resende RG, Correia-Silva J de F, Silva TA, Salomão UE, Marques-Silva L, Vieira ÉLM, et al. IL-17 Genetic and Immunophenotypic Evaluation in Chronic Graft-versus-Host Disease. Mediators Inflamm. 2014;2014:571231
Rongvaux-Gaïda D, Dupuis M, Poupon J, Djebrani-Oussedik N, Lemonnier C, Rieger F. High Response Rate and Corticosteroid Sparing with Arsenic Trioxide-Based First-Line Therapy in Chronic Graft-versus-Host Disease after Allogeneic Hematopoietic Stem Cell Transplantation. Transplant Cell Ther. 2022;S2666-6367(22)01469-5
Saidu NE, Bonini C, Dickinson A, et al. New approaches for the treatment of chronic graft-versus-host disease: current status and future directions. Front Immunol. 2020;11: 578314
Shiomi A, Usui T. Pivotal Roles of GM-CSF in Autoimmunity and Inflammation. Mediators Inflamm. 2015;2015:568543
Socié G, Ritz J. Current issues in chronic graft-versus-host disease. Blood. 2014;124(3):374-84
Staffas A, Burgos da Silva M, van den Brink MRM. The intestinal microbiota in allogeneic hematopoietic cell transplant and graft-versus-host disease. Blood. 2017;129(8):927-33
Stanley E, Lieschke GJ, Grail D, Metcalf D, Hodgson G, Gall JA, et al. Granulocyte/macrophage colony-stimulating factor-deficient mice show no major perturbation of hematopoiesis but develop a characteristic pulmonary pathology. Proc Natl Acad Sci USA. 1994;91(12):5592-6
Tugues S, Amorim A, Spath S, Martin-Blondel G, Schreiner B, De Feo D, et al. Graft-versus-host disease, but not graft-versus-leukemia immunity, is mediated by GM-CSF-licensed myeloid cells. Sci Transl Med. 2018;10(469):eaat8410
Yaniv I, Krauss AC, Beohou E, Dalissier A, Corbacioglu S, Zecca M, et al. Second Hematopoietic Stem Cell Transplantation for Post-Transplantation Relapsed Acute Leukemia in Children: A Retrospective EBMT-PDWP Study. Biol Blood Marrow Transplant. 2018;24(8):1629-42
Yoshizaki A, Yanaba K, Iwata Y, Komura K, Ogawa A, Akiyama Y, et al. Cell adhesion molecules regulate fibrotic process via Th1/Th2/Th17 cell balance in a bleomycin-induced scleroderma model. J Immunol Baltim Md 1950. 2010;185(4):2502-15
Zhao XY, Lv M, Xu LL, Qian X, Huang XJ. Donor Th17 cells and IL-21 may contribute to the development of chronic graft-versus-host disease after allogeneic transplantation. Eur J Immunol. 2013;43(3):838-50

## Claims

1. An *in vitro* method for the diagnosis, prognosis, stratification and/or monitoring of chronic graft-versus-host disease (cGVHD) in an individual who has received an allogeneic hematopoietic stem cell transplantation (allo-HSCT), comprising the steps of:
a) measuring the level of at least one cytokine selected from the group consisting of granulocyte-macrophage colony-stimulating factor (GM-CSF) and macrophage inflammatory protein (CCL4 or MIP-1β) in a biological sample from said individual,
b) optionally, measuring the level of at least one cytokine selected from the group consisting of Interleukin-1 alpha (IL-1α), Interleukin-17A (IL-17A), Interleukin-21 (IL-21) and chemokine (C-X-C motif) ligand 1 (CXCL1), in a biological sample from said individual,
c) comparing each of the levels measured in a) and, optionally, in b), to a reference value,
wherein levels in the biological sample higher than the reference values are indicative for the presence and/or aggravation of cGVHD.

2. The method of claim 1, wherein in step a) the levels of GM-CSF and CCL4 are measured.

3. The method of claim 1 or 2, wherein the reference value for each cytokine is a level of said cytokine measured in a previous sample from the individual.

4. The method of claim 3, wherein said previous sample from the individual has been taken prior to the allo-HSCT, and wherein a level of one or more of said cytokine(s) in the biological sample taken after the allo-HSCT higher than the reference value(s) indicates that the individual is likely to have or develop cGvHD.

5. The method of claim 1 or 2, wherein the reference value for each cytokine is a level of said cytokine measured in a cohort of healthy volunteers, and wherein a level of one or more of said cytokine(s) in the biological sample higher than the reference value indicates that the individual is likely to have or develop cGvHD.

6. The method of claim 3, wherein the reference value for each cytokine is a level of said cytokine measured in a previous sample from the individual, taken after the onset of cGVHD, and wherein a level of one or more of said cytokine(s) in the biological sample higher than the reference value is indicative for an aggravation of cGvHD.

7. The method of claim 6, wherein the individual receives a treatment of cGVHD and wherein a level of one or more of said cytokine(s) in the biological sample lower than the reference value is indicative for a response to the treatment.

8. The method of any one of claims 1 to 7, wherein the biological sample is a sample of blood, plasma or serum, preferably a serum sample.

9. The method of any one of claims 1 to 8, wherein the level of said cytokine(s) is measured by immunoassay.

10. A method for determining if an individual responds to a treatment of cGVHD, comprising performing the method according to any one of claims 1 to 9 at different time points, wherein a decrease in the level of one or more of said cytokines indicates that the individual responds to the treatment.

11. Use of GM-CSF and/or CCL4, alone or combined to one or several cytokines selected from the group consisting of IL-1α, IL-17A, IL-21 and CXCL1, as a biomarker for the diagnosis, prognosis, stratification and/or monitoring of cGVHD in a subject who has received an allo-HSCT.

12. A kit for the diagnosis, prognosis, stratification and/or monitoring of cGVHD, comprising reagents for measuring the level(s) of GM-CSF and/or CCL4 in a biological sample.

13. The kit of claim 12, further comprising reagents for measuring the level(s) of one or several cytokines selected from the group consisting of IL-1α, IL-17A, IL-21 and CXCL1.

14. The kit of claim 12 or claim 13, which comprises antibodies specifically recognizing the recited cytokine(s).

15. The kit of claim 14, wherein said antibodies are bound to a solid support.

16. The kit of claim 14 or 15, further comprising a biotinylated detection Ab.
